# EUROPEAN PATENT APPLICATION

(11) **EP 2 666 851 A1**
(43) Date of publication of application: **27.11.2013**
(21) Application number: 12736478.4
(22) Date of filing: 20.01.2012
(51) Int. Cl.: C12N 5/00, A01K 67/027, C12N 5/10, C12Q 1/02, G01N 33/15, G01N 33/50, G01N 33/68, C12N 15/09

(54) **GENE CAPABLE OF REGULATING OBESITY/INSULIN SENSITIVITY**

(30) Priority: 20.01.2011 JP 2011009375
(71) Applicant: Kyoto Prefectural Public University Corporation, Kyoto 602-8566 (JP)
(72) Inventor: IKEDA, Koji, Kyoto-shi Kyoto 602-8566 (JP); MATSUBARA, Hiroaki, Kyoto-shi Kyoto 602-8566 (JP); AKAKABE, Yoshiki, Kyoto-shi Kyoto 602-8566 (JP)
(74) Representative: Winter, Brandl, Fürniss, Hübner, Röss, Kaiser, Polte - Partnerschaft
(86) International application number: PCT/JP2012/051205
(87) International publication number: WO 2012/099243

(57) **Abstract**

This invention relates to a cell comprising a reporter gene under control of an ARIA gene promoter, the cell being used for searching for an agent for prevention or treatment of diseases attributed to reduced insulin sensitivity, for searching for an obesity-controlling substance, or for searching for an obesity-inducing substance.

## Description

### Technical Field

This application claims priority to Japanese Patent Application No. 2011-9375, filed on January 20, 2011, the entire contents of which are hereby incorporated by reference.

The present invention relates to a method for screening an agent for prevention and/or treatment of obesity or metabolic syndrome, an obesity-inducing substance, and a nonhuman animal with a predisposition to obesity, and more specifically to a method for screening based on the expression level of an ARIA gene.

Further, the present invention relates to use of the ARIA gene for searching for an agent for prevention or treatment of insulin resistance and an obesity-inducing substance, and relates to an animal model.

### Background Art

Insulin resistance refers to disorders caused by increased insulin resistance. It is known that insulin resistance are closely associated with adult diseases, such as type 2 diabetes, hyperlipidemia/hepatic steatosis, obesity, hypertension, and arteriosclerosis.

For the purpose of analyzing the mechanism of such pathological conditions and developing a therapeutic agent thereof, various mouse models of disease have been developed. For example, a mouse model of diabetes is created by deleting a gene that appears to be associated with diabetes, and used for analyzing the mechanism of the gene or for screening an antidiabetes agent. In NPL 1, major mouse models of diabetes are enumerated.

The present inventors have identified a gene that is significantly expressed in vascular endothelial cells by using a signal sequence trap method. The gene was named ARIA, and published an article about ARIA (NPL 2).

It is desirable that farm animals, such as swine, cattle, and fowls, rapidly gain weight when fed a feed containing the same number of calories. Thus, farm animals with a predisposition to obesity, an obesity-inducing substance, and a search method therefore have been desired.

### Citation List

### Non-patent Literature

NPL 1: Bessatsu Igaku No Ayumi, Ishiyaku Publishers, Inc., pp113-117, 2004
NPL 2: Proc Natl Acad Sci USA 106(20), 2009

### Summary of Invention

### Technical Problem

An object of the present invention is to identify a gene associated with obesity and insulin sensitivity, and to establish a screening system to search for an agent for prevention or treatment of these disorders, an obesity-inducing substance, and a nonhuman animal with a predisposition to obesity, such as farm animals and fish.

### Solution to Problem

The present inventors have identified a gene that is significantly expressed in vascular endothelial cells by using a signal sequence trap method. The gene was named ARIA, and published an article about ARIA (NPL 2).

Since reporting on the ARIA gene, the present inventors have discovered that ARIA is significantly expressed also in adipose tissues, such as white adipose and brown adipose. The present inventors produced ARIA gene knockout mice, and found that when fed a high-fat diet, the ARIA gene knockout mice evidently gained less weight than wild-type mice and showed a decrease in the amount of visceral fat, while having high insulin sensitivity even on a normal diet; accordingly, the inventors completed the present invention.

As described hereinbelow, the present invention relates to: methods for screening an agent for prevention or treatment of a disease attributed to a decrease in insulin sensitivity, such as obesity or metabolic syndrome; use of the ARIA gene to search for an agent for prevention and treatment of a disease attributed to a decrease in insulin sensitivity, an obesity-controlling substance, an obesity-inducing substance or a nonhuman animal with a predisposition to obesity; animal models; and cells.
Item 1. A cell for searching for an agent for prevention or treatment of a disease attributed to a decrease in insulin sensitivity or an obesity-controlling substance, or for searching for an obesity-inducing substance, the cell comprising a reporter gene under control of an ARIA gene promoter.
Item 2. A cell characterized by an overexpression of ARIA by introduction of an ARIA gene.
Item 3. The cell according to Item 2, wherein the overexpression of ARIA enhances binding between ARIA and PTEN.
Item 4. A cell for searching for an agent for prevention or treatment of a disease attributed to a decrease in insulin sensitivity or an obesity-controlling substance, or for searching for an obesity-inducing substance, the cell capable of detecting binding between an ARIA protein and a PTEN protein.
Item 5. The cell according to Item 4, wherein the ARIA protein and the PTEN protein each have been fused with a protein that produces or quenches fluorescence when the ARIA and PTEN proteins interact with each other.
Item 6. An animal model of a disease attributed to a decrease in insulin sensitivity, the animal model characterized by an overexpression of ARIA gene.
Item 7. The animal model according to Item 6, which is a transgenic mouse.
Item 8. A method for screening an agent for prevention or treatment of a disease attributed to a decrease in insulin sensitivity, or an obesity-controlling substance, an obesity-inducing substance or a nonhuman animal with a predisposition to obesity, the method using an increase and/or a decrease in an expression level of an ARIA gene as an index.
Item 9. The method for screening an agent for prevention or treatment of a disease attributed to a decrease in insulin sensitivity or an obesity-controlling substance according to Item 8, the method comprising:
   contacting a candidate substance with the cell according to any one of Items 1 to 5; and
   determining the candidate substance to be an agent for prevention or treatment of a disease attributed to a decrease in insulin sensitivity or an obesity-controlling substance when the expression level of the ARIA gene is decreased.
Item 10. The method for screening an obesity-inducing substance according to Item 8, the method comprising:
   contacting a candidate substance with the cell according to any one of Items 1 to 5;
   determining the candidate substance to be an obesity-inducing substance when the expression level of the ARIA gene is increased.
Item 11. A method for screening an agent for prevention or treatment of a disease attributed to a decrease in insulin sensitivity, or an obesity-controlling substance, or an obesity-inducing substance, the method comprising:
   contacting a candidate substance with the animal model according to Item 6 or 7; and
   evaluating a change in insulin sensitivity.
Item 12. A method for screening a nonhuman animal with a predisposition to obesity, the method comprising the steps of:
   measuring an expression level of an ARIA gene in a nonhuman animal; and
   determining the nonhuman animal to have a predisposition to obesity when the expression level of the ARIA gene is high.
Item 13. An animal model for searching for an obesity-inducing substance, the animal model having an ARIA gene knocked out.
Item 14. A method for detecting a disease attributed to a decrease in insulin sensitivity, the method comprising:
   detecting an expression level of an ARIA protein in a subject sample; and
   evaluating insulin sensitivity by using the expression level of the ARIA protein as an index.
Item 15. The method according to Item 14, wherein the sample is selected from the group consisting of blood, blood serum, blood plasma, urine, lachrymal fluid, and saliva, and preferably blood plasma or blood serum.
Item 16. Use of an expression product of an ARIA gene to search for an agent for prevention or treatment of a disease attributed to a decrease in insulin sensitivity, an obesity-controlling substance or an obesity-inducing substance, or a nonhuman animal with a predisposition to obesity.
Item 17. The use of an expression product of an ARIA gene according to Item 16, wherein the expression product of the ARIA gene is mRNA or ARIA protein.
Item 18. The cell according to any one of Items 1 to 5, the animal model according to Item 6 or 7, the method for screening according to any one of Items 8 to 11, or the use according to Item 16 or 17, wherein the disease attributed to a decrease in insulin sensitivity is obesity, type 2 diabetes, or metabolic syndrome.

### Advantageous Effects of Invention

In the present invention, the involvement of the ARIA gene in obesity and insulin sensitivity has been elucidated for the first time. Thus, searching for a substance that suppresses ARIA gene expression enables the screening of an agent for prevention or treatment of diseases attributed to obesity or reduced insulin sensitivity (elevated insulin resistance) and an obesity-controlling substance. Likewise, searching for a substance that promotes ARIA gene expression enables the screening of an obesity-inducing substance and a nonhuman animal with a predisposition to obesity.

### Brief Description of Drawings

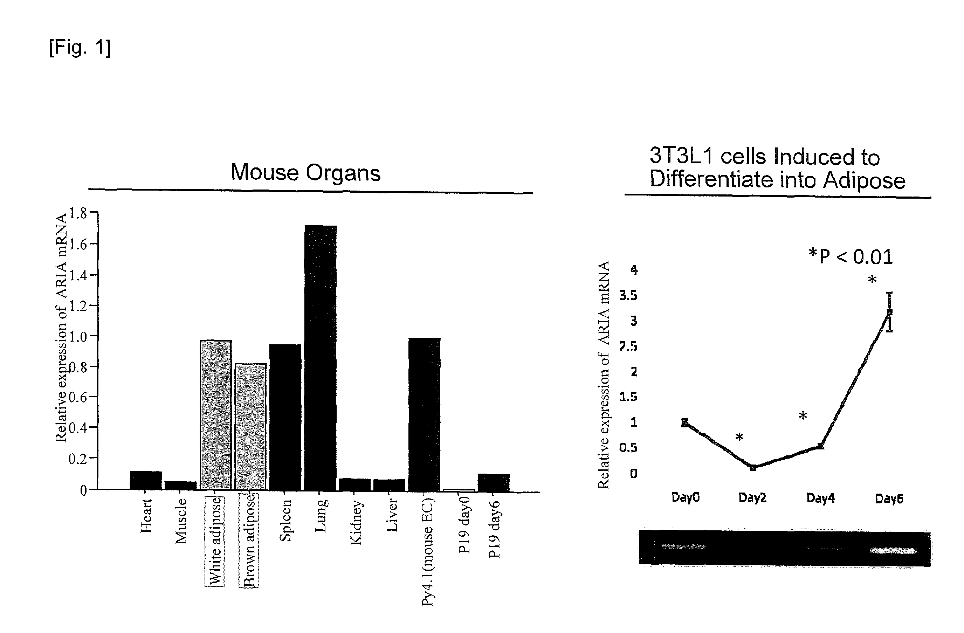

Fig. 1(A) shows that ARIA was significantly expressed in white adipose and brown adipose. The vertical axis denotes the relative expression level of ARIA mRNA when Py4.1 ARIA mRNA expression level is 1, and the horizontal axis denotes individual organs. Fig. 1(B) shows a change in ARIA mRNA expression level in 3T3L1 cells that were differentiation-induced adipose cells. The vertical axis denotes the relative expression level of ARIA mRNA when the expression level of ARIA mRNA before differentiation induction (day 0) is 1, and the horizontal axis denotes the cumulative days since differentiation induction was started. An electrophoretogram corresponding to the expression level of ARIA mRNA is also shown below the figure.
Fig. 2A shows the obesity level of ARIA knockout mice on a normal diet. Between ARIA knockout mice (KO) and wild-type mice (WT), there was little difference in body weight and fat mass, when fed a normal diet.
Fig. 2B shows the obesity level of ARIA knockout mice on a high-fat diet. Under the high-fat diet condition, ARIA knockout mice (KO) showed a significantly lower increase rate of body weight and had a significantly lower subcutaneous fat mass than wild-type mice (WT).
Fig. 3 shows the results of an oral glucose tolerance test (OGTT) (A) and an insulin tolerance test (ITT) (B) performed on ARIA knockout mice and wild-type mice. It was found that ARIA knockout mice had higher insulin sensitivity than wild-type mice even when fed a normal diet.
Fig. 4A shows that insulin signal transduction was increased in liver (A), and skeletal muscle and white adipose (B) of ARIA knockout, as compared to wild-type mice.
Fig. 4B shows that insulin signal transduction was increased in liver (A), and skeletal muscle and white adipose (B) of ARIA knockout, as compared to wild-type mice.
Fig. 5 shows that ARIA binds to PTEN.
Fig. 6 is a diagram illustrating the mechanism of involvement of ARIA in an increase in insulin sensitivity. ARIA binds to PTEN, which is an endogenous molecule that inhibits insulin signaling, causing PTEN to localize immediately under the plasma membrane and be activated, thereby suppressing downstream signaling of insulin receptors (PI3K-Akt). We presume that control over PI3K-Akt signaling is lost in ARIA knockout mice, which are ARIA null, and this causes an increase in insulin sensitivity.
Fig. 7 shows a cell line that is capable of detecting the binding between ARIA and PTEN as a fluorescent signal. The cell that glows green indicates binding between ARIA and PTEN.
Fig. 8 shows that cell lines that stably express ARIA were established by using CHO cells that do not express ARIA, and it was confirmed that PTEN was increased by ARIA in the membrane fraction.
Fig. 9 shows that soluble ARIA extracellular domains were released in the culture supernatant of CHO cells that expresses ARIA (A). Further, when the ARIA expression level was changed by the amount of plasmid used for gene introduction, the higher the ARIA expression level was, the higher the amount of the soluble ARIA extracellular domains became in the culture supernatant (B). Thus, we assume that ARIA expression level in cells can be predicted by measuring the expression level of the soluble ARIA extracellular domains.
Fig. 10 shows the results of a western blot assay of the cell lines that stably expressed ARIA. Stable line #6, which showed the highest ARIA expression, was used in subsequent experiments.
Fig. 11 shows that ARIA knockout mice loaded with a high-fat diet exhibited an increase in oxygen consumption and a decrease in respiratory quotient as compared to wild-type mice in spite of there being no difference in activity level.
Fig. 12 shows that ARIA knockout mice on a high-fat diet exhibited an increased heat production capacity as compared to wild-type mice.
Fig. 13 shows that the mice that overexpressed ARIA specifically in vascular endothelial cells displayed significantly reduced ischemia-induced angiogenesis.
Fig. 14 shows that the mice that overexpressed ARIA specifically in vascular endothelial cells were more susceptible to gaining weight, and showed worsened glucose tolerance and insulin sensitivity after being fed a high-fat diet, as compared to wild-type mice.
Fig. 15 shows that the mice which overexpressed ARIA specifically in vascular endothelial cells on loaded with a high-fat diet displayed a depressed heat production capacity as compared to wild-type mice.

### Description of Embodiments

ARIA has been heretofore known to be abundantly expressed in vascular endothelial cells, whereas, ARIA is not known to be associated with obesity, an increase in insulin resistance, a decrease in insulin sensitivity, and, for example, diabetes.

Since we revealed that ARIA is closely associated with obesity and insulin sensitivity, a search for an obesity-inducing substance or an obesity-controlling substance, an agent for enhancing insulin sensitivity, and nonhuman animals, such as farm animals and fish, with predisposition to obesity can be searched for according to the present invention.

The term "ARIA" as used herein stands for "apoptosis regulator through modulating IAP expression." The ARIA gene is known and registered in several databases. Examples of GenBank accession numbers for the ARIA gene are EU025066 (human), EU025067 (mice), and the like. The ARIA gene can be suitably selected according to the animal cell and/or the animal species to be used. It is preferable to introduce a foreign ARIA gene that originated from the same species as that of a cell into which the foreign ARIA gene is introduced, and it is preferable to introduce a human ARIA gene into a human cell. An allele of an ARIA gene or a modified gene that has ARIA functions may be used.

ARIA promoter sequences are also registered in several databases, and ARIA promoters that originate from the cells of different species are thus obtained from such databases.

The term "obesity" as used herein refers to obesity caused by the intake of a high-calorie diet.

As used herein, "disease attributed to a decrease in insulin sensitivity" includes diabetes (in particular, type 2 diabetes), abnormal glucose tolerance, adiposity, hyperlipidemia, arteriosclerosis, hypertension, cardiac diseases, metabolic syndrome, and the like.

The term "obesity-controlling substance" refers to a substance that controls obesity by suppressing the expression, function, and activity of ARIA, and particularly refers to a substance that controls obesity caused by the intake of a high-calorie diet. The obesity-controlling substance may be taken with meals, or separately from meals. The obesity-controlling substance may be in a pharmaceutical form, or may be taken as food or a supplement.

The term "obesity-controlling substance" refers to a substance that induces obesity by activating the expression, function, and activity of ARIA, and particularly refers to a substance that induces obesity when a high-calorie diet is consumed. Such a substance can efficiently fatten farm-raised fish and animals, such as cattle, swine, fowls, and sheep, to allow them to be quickly shipped to market.

A nonhuman animal with a predisposition to obesity refers to an animal that produces a large amount of a product of ARIA expression, such as ARIA mRNA, or ARIA protein. When an animal produces a higher amount of the ARIA expression product than the benchmark, the animal is determined to be a nonhuman animal with a predisposition to obesity. The amount of an ARIA expression product can be measured with an immunoassay that uses an antibody, e.g., ELISA, EIA, or immunochromatography, to measure the amount of ARIA protein in a sample of blood, blood serum, blood plasma, urine, lachrymal fluid, or saliva, and preferably in a sample of blood plasma or blood serum. As a nonhuman animal with a predisposition to obesity, farm animals, such as cattle, swine, fowl, sheep and the like, and farmed fish and shellfish (eels, sea bream, scallops, oysters, yellowtail, etc.) are particularly useful.

In one embodiment of the present invention, the cells used for screening are those into which the ARIA gene has been introduced in an expressible form, thereby overexpressing ARIA. The cells may be either prokaryotic cells such as bacteria, or eukaryotic cells such as yeast, plant cells, or animal cells, and preferably eukaryotic cells, and more preferably animal cells. The animal cells may be those of mammals such as humans, mice, rats, rabbits, dogs, cats, monkeys, cows, horses, sheep, goats, and pigs; fowl, such as hens, and ducks; amphibians, such as frogs; reptiles, such as newts; fish and shellfish; or insects; preferably mammal cells, and most preferably human cells. A candidate compound is added to a medium containing cells that overexpress ARIA, and screening for an increase or decrease in the expression level of ARIA is performed so that an obesity-inducing substance (when the ARIA expression level is increased) or an obesity-controlling substance (when the ARIA expression level is decreased) can be searched for. The ARIA expression level may be measured by measuring the amount of mRNA or protein using a northern blotting, western blotting or immunological assay such as ELISA, and preferably by measuring the expression level of reporter genes under the control of ARIA promoters. Because ARIA knockout animal models are resistant to obesity even when fed a high-fat diet, screening for an obesity-inducing substance is performed by evaluating the progress of obesity after a mixture of a candidate compound with a high-fat diet is administered to the animals, as necessary. When added to the feed for farm animals, such as swine, cattle, sheep, fowl, boars, and the like, an obesity-inducing substance can cause an increase in the body weight of the animals within a short period of time or with a small amount of feed. By evaluating the ARIA expression level, a breed of farm animal having predisposition to obesity can be revealed; in humans, it can be assessed whether one has a predisposition to obesity-related diseases, such as metabolic syndrome.

ARIA transgenic animal models readily become obese, particularly when fed a high-fat diet. Thus, by administering a candidate compound to the animals and evaluating the obesity resistance of the animals, an obesity-controlling substance can be screened for.

Reporter gene examples include: lacZ; fluorescent proteins such as luciferase (Vargula hilgendorfii, fireflies, Renilla reniformis, Aequorea victoria, aequorin), GFP, YFP, BFP, CFP, DsRED, or RFP; chloramphenicol acetyltransferase; etc.

The ARIA gene is typically introduced into a cell in the form of an expression vector that comprises one or more functionally linked gene expression regulatory sequences. The ARIA gene may comprise sequences including introns, for example. The "gene expression regulatory sequence" refers to a nucleic acid sequence that regulates the expression of a functionally linked nucleic acid sequence to be expressed. The gene expression regulatory sequence is functionally linked to the nucleic acid sequence to be expressed when regulating and modulating transcription of the nucleic acid sequence, and also in the case of regulating and modulating translation of the nucleic acid sequence. Therefore, the gene expression regulatory sequence can comprise a suitable promoter, enhancer, transcriptional termination factor, initiation codon (ATG) preceding a protein coding gene, splicing signal for introns, maintenance of a correct reading frame that enables proper translation of mRNA, and termination codon.

In the present invention, a protein, which provides energy transfer, e.g., FRET, can be bound to each ARIA gene and PTEN gene so that a signal, such as fluorescence, is generated when the binding between ARIA and PTEN occurs; such binding being intervened by adequate spacers as necessary. Examples of protein combinations are shown below:
- Luciferase and a fluorescent protein (GFP, YFP, etc.)
- mKG that is divided into half
- Fluorescein/bis-thiobarbiturate trimethine oxonol;
- Natural fluorescent protein/bis-thiobarbiturate trimethine oxonol;
- Natural fluorescent protein/bis-thiobarbiturate pentamethine oxonol;
- Coumarin/bis-thiobarbiturate trimethine oxonol;
- Coumarin/bis-thiobarbiturate pentamethine oxonol;
- Bis-thiobarbiturate trimethine oxonol/Texas red;
- Bis-thiobarbiturate trimethine oxonol;/resorufin;
- Bis-thiobarbiturate trimethine oxonol/Cys5;
- Bis-thiobarbiturate trimethine oxonol/bis-thiobarbiturate pentamethine oxonol;
- Texas red/bis-thiobarbiturate pentamethine oxonol;
- NBD/bis-thiobarbiturate trimethine oxonol;
- NBD/bis-thiobarbiturate pentamethine oxonol.

In one embodiment of the present invention, a combination of an energy donor and its acceptor that can be expressed intracellularly is preferable, and it is preferable that the energy donor and the acceptor are composed of proteins or peptides. Another preferable embodiment of the present invention is a cell line into which the ARIA gene and the PTEN gene are introduced after each of the genes has been bound to a half-cut mKG, for evaluating the degree of binding.

The binding of ARIA and PTEN leads to obesity, particularly with a high-fat diet. Thus, by adding a candidate substance to a culture containing cells that have expressed the combination of the energy donor and acceptor and detecting a change in the binding of ARIA and PTEN through the energy transfer level, it is possible to evaluate whether the candidate substance induces obesity (when energy transfer is increased) or controls obesity (when energy transfer is decreased); this accordingly enables screening for both an obesity- inducing substance and an obesity-controlling substance by using a single cell system.

ARIA knockout mouse models produced by the present inventors exhibit an increase in insulin sensitivity with no other defects, and a medicinal substance that inhibits ARIA expression is considered to have no serious adverse effect on the mouse models. When the ARIA knockout mouse models are fed a high-fat diet, an increase in body weight is suppressed. On the other hand, when the ARIA knockout mouse models are fed a normal diet, the body weight and adipose mass are not significantly affected. Therefore, the substance that suppresses ARIA has ideal properties that prevent obesity while maintaining a healthy weight.

ARIA transgenic animal models produced by the present inventors exhibit a decrease in insulin sensitivity. A decrease of vascular density in brown adipose, and a reduction in the expression of gene clusters associated with heat production and fatty acid oxidation are also observed. ARIA may be expressed throughout the body, and may be expressed in a site-specific manner, for example, in vascular endothelial cells or adipose cells (white adipose cells or brown adipose cells). The ARIA transgenic animal models are animal models of obesity caused by high fat diets. An obesity-controlling substance obtained through screening by using the animal models of obesity does not significantly affect the body weight and adipose mass when the animal models of obesity are fed a normal diet, thereby having ideal properties that prevent obesity while maintaining a healthy weight.

### Examples

Hereinafter, the present invention is described in more detail with reference to the Examples, but the present invention is not limited to the Examples.

### Example 1

(1) ARIA expression in individual organs of mice Total RNAs were extracted from the individual organs of mice (heart, muscle, white adipose, brown adipose, lung, kidney, liver, and Py4.1 (mouse EC)). Thereafter, cDNAs were created using reverse transcriptase, and quantitative analysis of ARIA gene expression was conducted by a quantitative PCR method using the cDNAs as a template. The results are shown on the left hand side of Fig. 1. It was confirmed that ARIA was highly expressed also in adipose tissues, such as white adipose and brown adipose.

### (2) Induction to differentiate into adipose cells

3T3-L1 mouse fibroblast cells were induced to differentiate into adipose cells by using insulin, dexamethasone, and pioglitazone. Total RNAs were extracted from the cells prior to the start of induction (Day 0) and on Days 2, 4, and 6 after the start of induction, and quantitative analysis of ARIA gene expression was conducted in the same manner as in Section (1) above. The results are shown in Fig. 1(B). As seen from Fig. 1 (B), the expression level of ARIA was increased in accordance with the differentiation into adipose cells.

From the results, the present inventors inferred that ARIA is somehow involved in insulin resistance.

### Example 2

### (1) Production of ARIA knockout mice

The procedure for producing ARIA knockout mice is described below.
(i) A targeting vectors was constructed by replacing ARIA exons 1 to 3 with a Neo-cassette, and it was electroporated into C57BL6-derived ES cells.
(ii) The ES cells that underwent homologous recombination were injected into mouse blastocysts, and the obtained blastocysts were transplanted into the uteri of host mice.
(iii) F0 heterozygous knockout mice were produced from chimera mice in which genetically modified ES cells differentiated into germ-line cells. Homozygous knockout mice were then produced by crossing the heterozygous knockout mice.

### (2) Evaluation of ARIA knockout mice

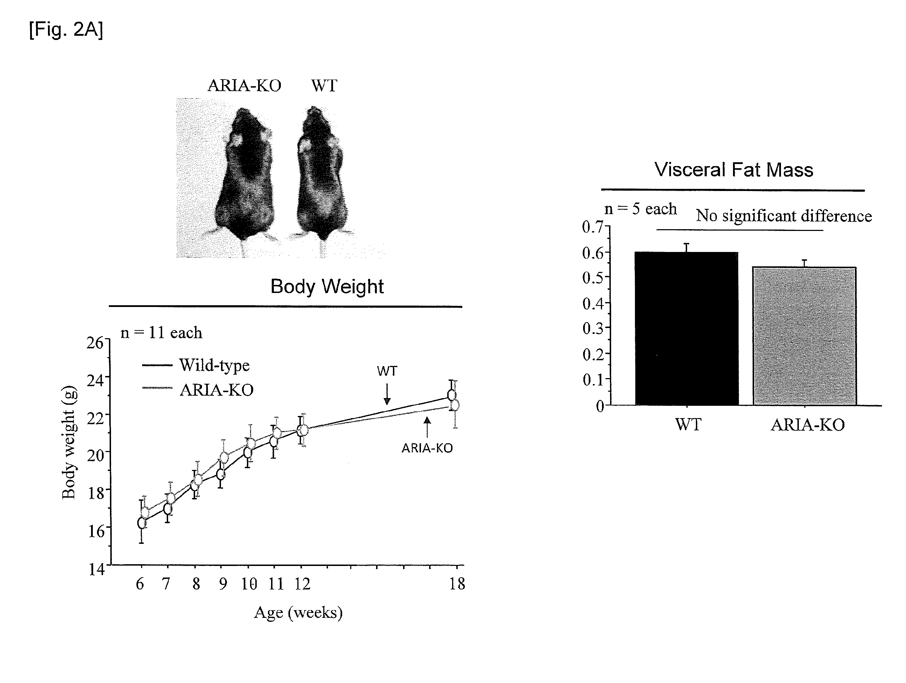

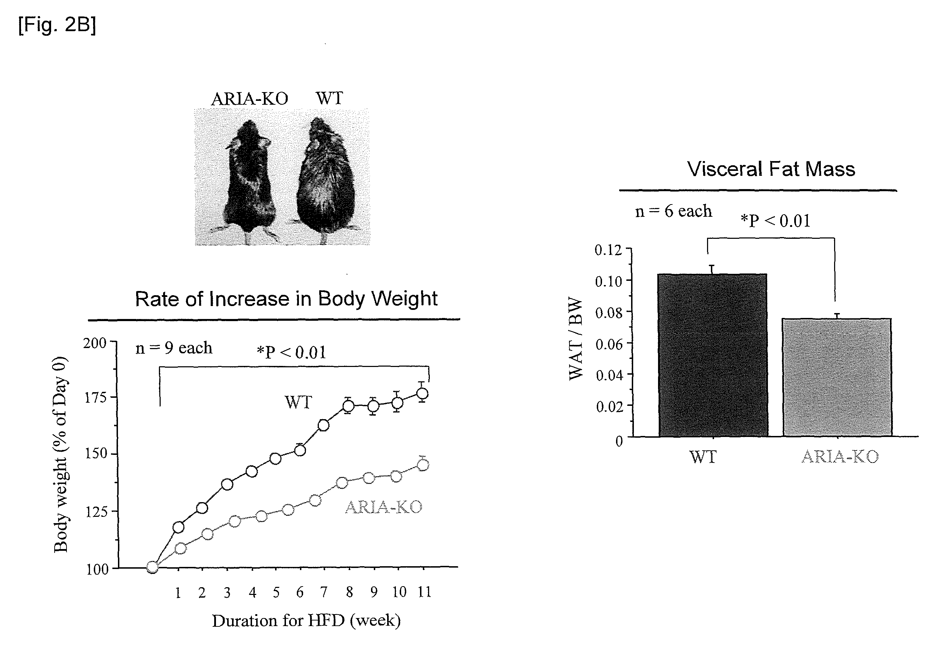

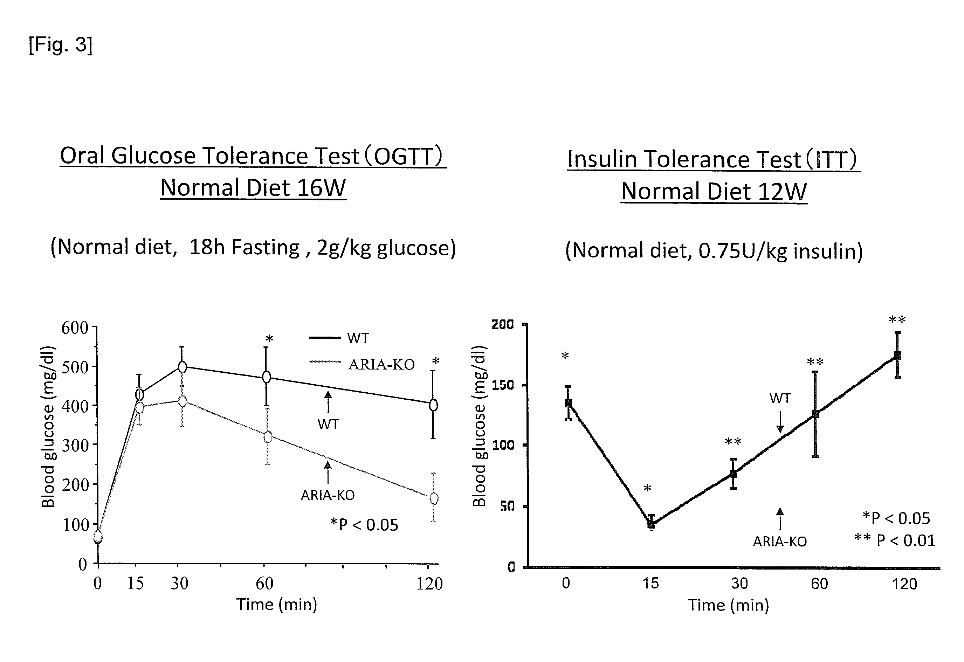

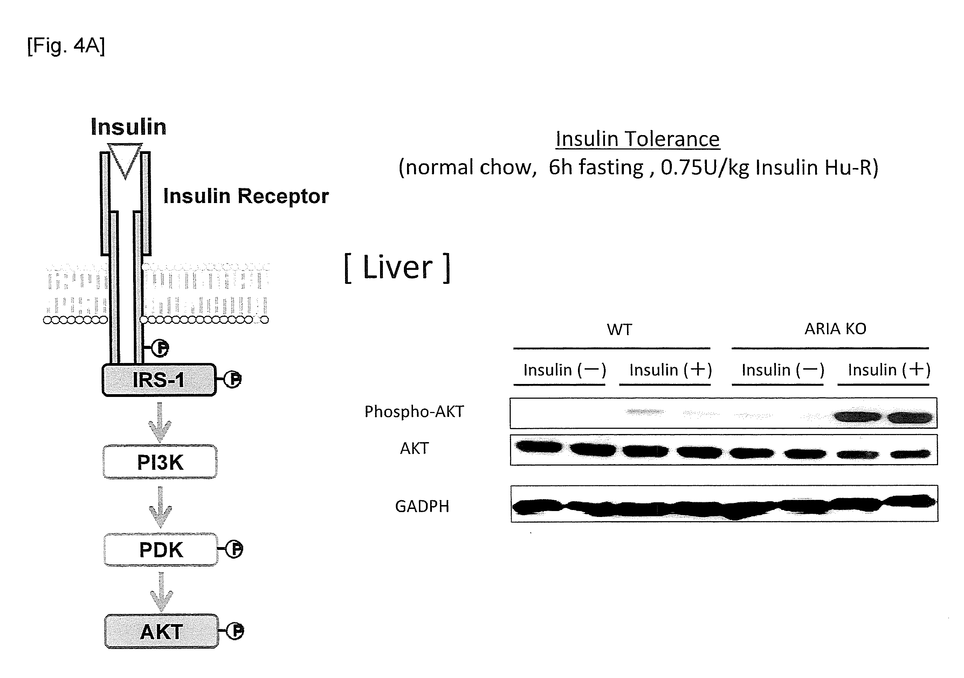

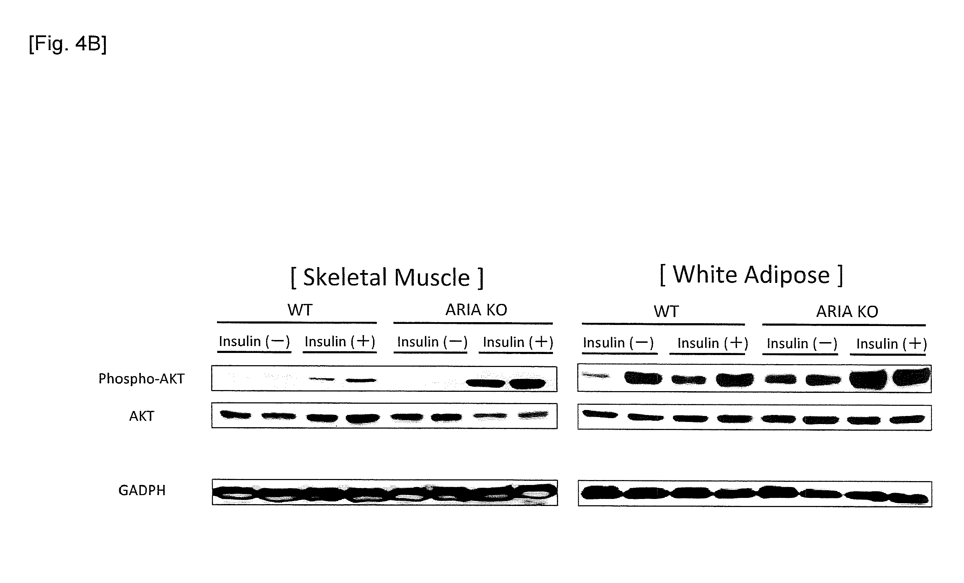

When ARIA knockout mice were fed a normal diet, there was no significant difference between wild-type mice and ARIA knockout mice in body weight and adipose mass (Fig. 2 (A)). In contrast, when loaded with a high-fat diet (HFD-60 produced by Oriental Yeast Co., Ltd.; a high-fat feed, 60% calories of which is from fat), the body weight of ARIA knockout mice clearly increased less than that of wild-type mice, and a decrease in the amount of visceral fat was observed (Fig. 2 (B)). Further, an oral glucose tolerance test (OGTT) and an insulin tolerance test (ITT) revealed that ARIA knockout mice had higher insulin sensitivity than wild-type mice even when fed a normal diet (Fig. 3). Specifically, 2 g/kg of glucose was administered through a gastric tube and 0.75 U/kg of insulin was administered through subcutaneous injection, respectively, and blood glucose was measured by conducting blood collection from the retro-orbital sinus over time. In addition, it was found that intracellular insulin signal transduction was noticeably increased in the liver, white adipose, and skeletal muscle, which are insulin target organs, in ARIA knockout mice (Fig. 4A, 4B). Specifically, 0.75 U/kg of insulin was administered to the mice by subcutaneous injection; 10 minutes later, the liver, white adipose, and skeletal muscle were harvested. The harvested tissues were immediately lysed on ice in a protein lysis solution containing SDS. After insoluble fractions were removed by centrifugation, electrophoresis was performed using an SDS-PAGE gel. After the proteins on the gel were transferred onto a nitrocellulose membrane, immunoblotting was performed using a specific antibody, followed by quantitative analysis of the phosphorylation of Akt, which is a downstream molecule of insulin signaling. As a result, ARIA knockout mice exhibited noticeably increased intracellular insulin signal transduction in all of the liver, white adipose, and skeletal muscle, as compared to wild-type mice.

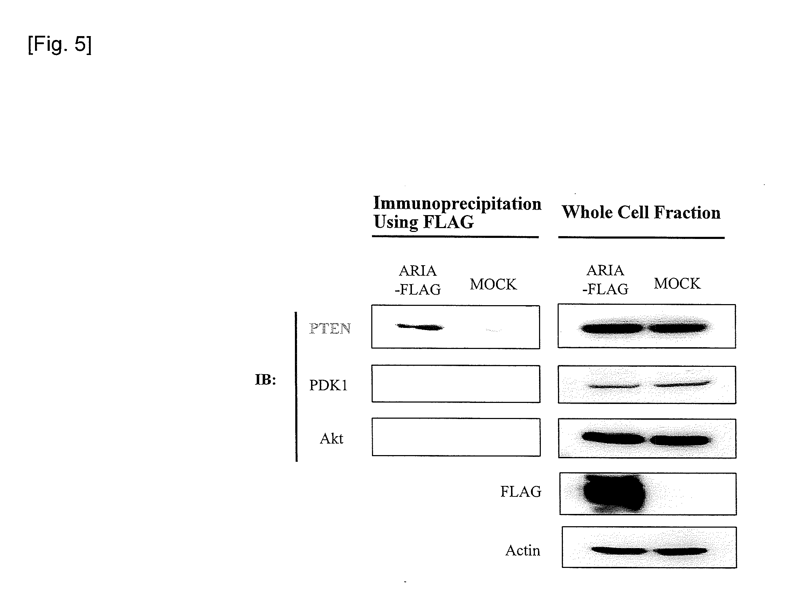

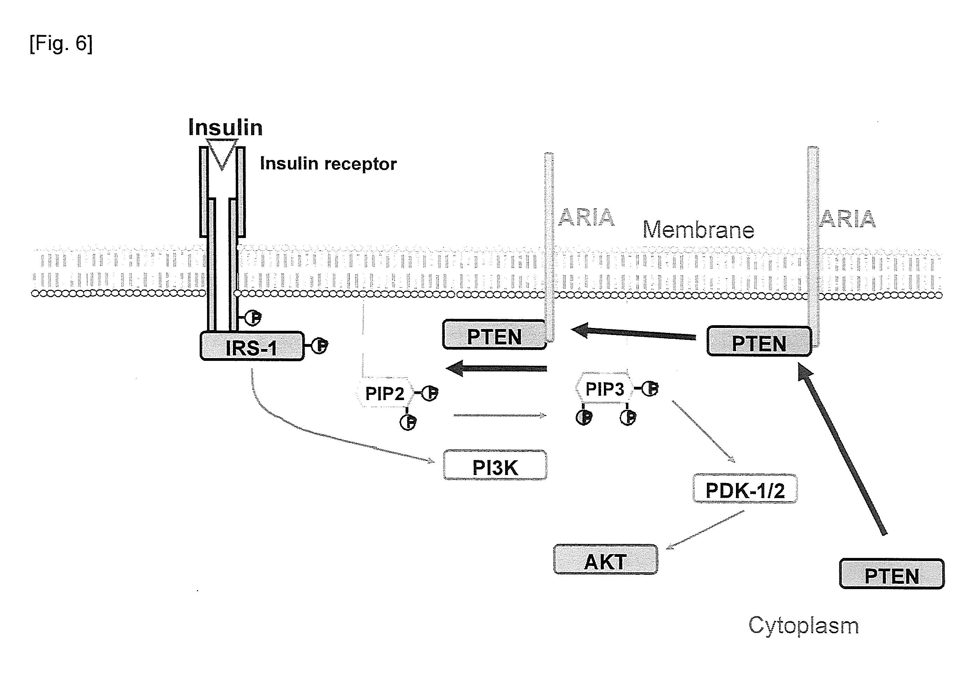

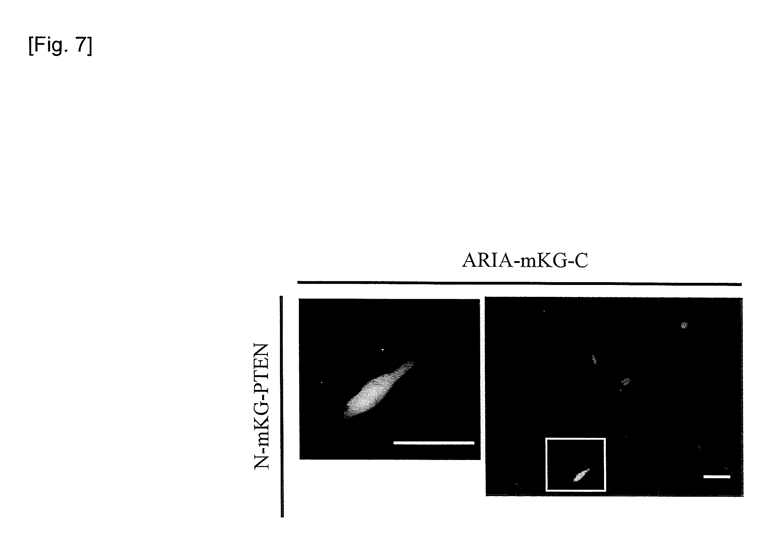

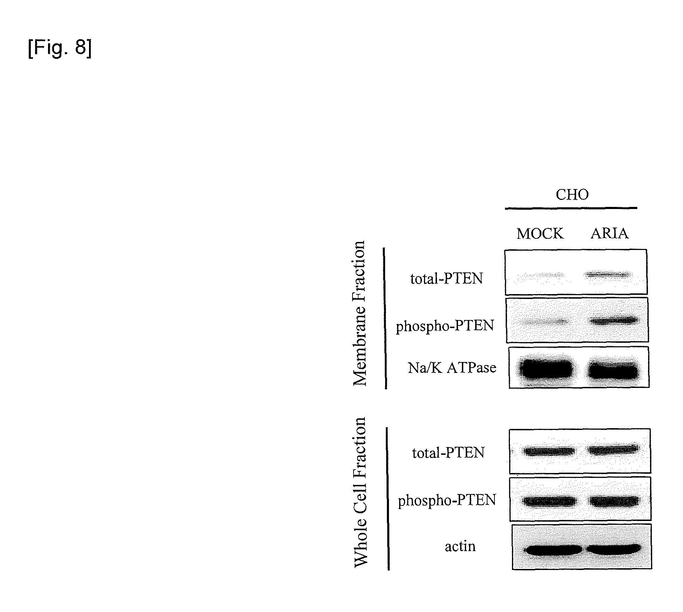

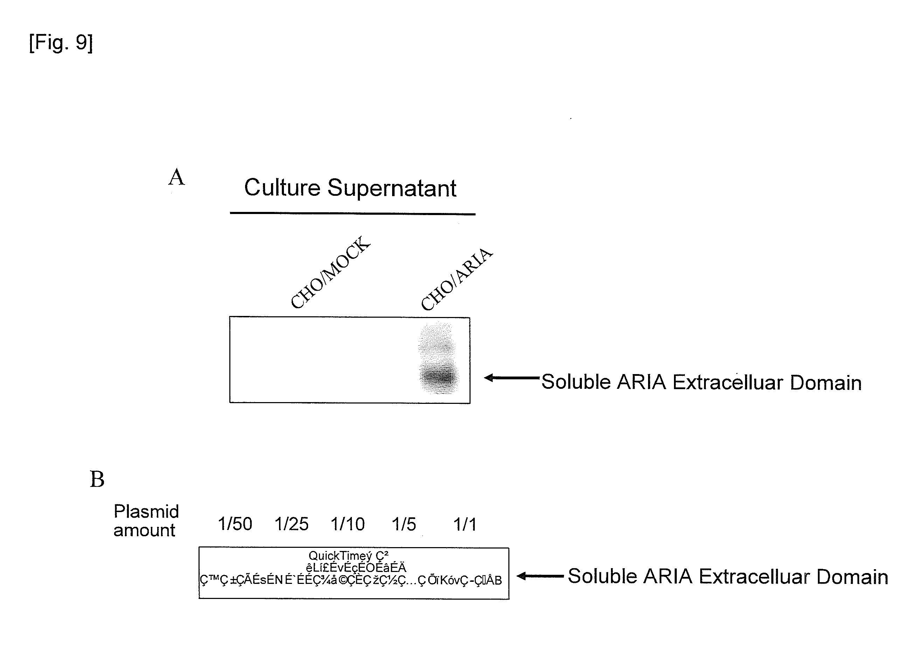

Moreover, an analysis using cultured cells has revealed that ARIA binds to Phosphatase and Tensin Homolog Deleted from Chromosome 10 (PTEN) (Fig. 5). Specifically, ARIA fused with the FLAG-tag was expressed in py4.1 cells, which are cell lines of mouse vascular endothelial cells, by a transfection method. The cells were lysed in a cell lysis solution containing 1% Triton-X100, and insoluble fractions were removed by centrifugation. Immunoprecipitation of ARIA-FLAG was performed using an anti-FLAG antibody and protein G. Precipitated proteins were subjected to electrophoresis on SDS-PAGE gel and then transferred onto a nitrocellulose membrane, followed by immunoblotting using a specific antibody. PTEN is a phosphatase that antagonizes phosphoinositide 3-kinase (PI3K), which is a downstream molecule of insulin signaling. PTEN inhibits the function of PI3K by dephosphorylating membrane phospholipid (PIP3) that has been phosphorylated by PI3K, thus converting PIP3 back to PIP2. Therefore, it is known that PTEN is cannot exert its function unless it is located in the vicinity of the plasma membrane. Because ARIA is a membrane protein that is present on the plasma membrane, we speculate that ARIA localizes PTEN, which negatively regulates insulin signaling, in the vicinity of the plasma membrane to activate PTEN, thereby inhibiting insulin signal transduction (Figs. 6 and 8). We presume that ARIA deletion results in a loss of regulation over insulin signal transduction that occurs through insulin stimulation; accordingly, ARIA knockout mice show high insulin sensitivity. Therefore, a substance that suppresses ARIA is expected to become an entirely new pharmaceutical agent for diseases attributed to obesity or reduced insulin sensitivity, and/or metabolic syndrome, and screening of such a substance for those diseases can be performed by measuring/detecting the ARIA expression level and the binding between ARIA and PTEN. The present invention explains the rationale for why an ARIA function inhibitor is useful as an agent for treating obesity, insulin resistance, and metabolic syndrome. ARIA functional inhibition refers to the inhibition of binding between ARIA and PTEN. The present inventors have established a CHO cell line as an assay system to screen compounds for inhibiting binding between ARIA and PTEN, the CHO cell line expressing recombinant proteins in which ARIA and PTEN are fused with reporter molecules made by dividing Kusabira-Green (mKG), a fluorescent substance, in half (the first half named mKG-N and the second half named mKG-C, respectively). When ARIA and PTEN bind to each other, the half-divided mKGs are reconstituted, thereby generating green fluorescence (Fig. 7). When a compound that inhibits the binding between ARIA and PTEN acts upon the cell, the ARIA and PTEN binding is inhibited; as a result, the half-divided mKGs are not reconstituted, and the fluorescence intensity decreases. Since fluorescence intensity can be easily and quickly measured with a plate reader, a large-scale preliminary screening can be performed with this assay system. Further, the present inventors have produced a stable expression cell line (CHO/ARIA) that constantly expresses ARIA in CHO cells, which do not originally express ARIA. After plasma membrane fractions were extracted by an ultracentrifugal method, the expression level of PTEN localized to the membranes was examined with an immunoblotting assay. In comparison, PTEN localized to the plasma membranes clearly increased more in CHO/ARIA cells than in control cells (Fig. 8). The increase is presumed to be attributed to PTEN that was bound to ARIA on the plasma membranes. Thus, when a test medicinal substance that inhibits the binding between ARIA and PTEN is added, PTEN in the plasma membrane fractions of CHO/ARIA should decrease. This assay system enables evaluation of the inhibition of ARIA and PTEN binding more quantitatively, and in a real-time manner, and is therefore ideally suited for a secondary screening of candidate substances that have been found to be positive in a preliminary screening. More specifically, an assay system using the cell line established by the present inventors allows preliminary and secondary screening for an agent that prevents and/or treats obesity and/or metabolic syndrome, the assay being characterized in that interaction between ARIA and PTEN is used as an index. It is known that many of the membrane proteins are cleaved in specific/non-specific response and that a portion of the extracellular domain is liberated. Likewise, it was confirmed that a portion of the extracellular domain of ARIA was liberated into a culture supernatant (Fig. 9). The results strongly implicate the possibility that a portion of the extracellular domain of ARIA is released into blood, and it is highly likely that change in the ARIA expression level can be evaluated simply by measuring ARIA in the blood. Accordingly, ARIA can be a new blood biomarker for diseases attributed to obesity and a decrease in insulin sensitivity, and/or metabolic syndrome.

### Example 3: Establishment of cell line

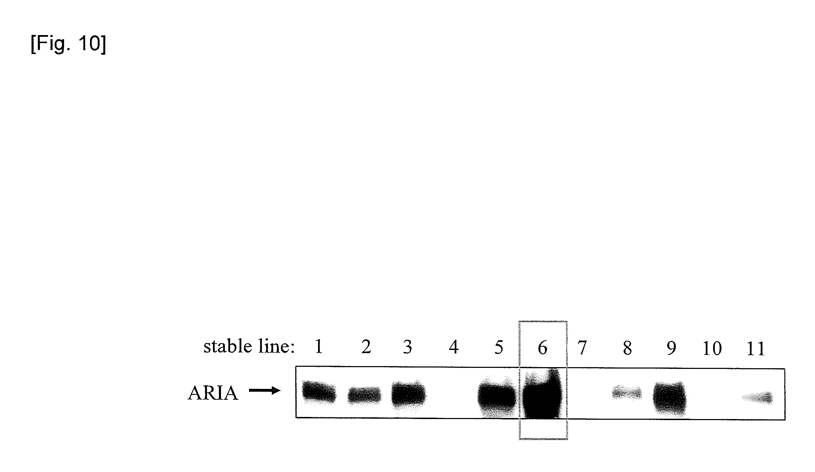

CHO-K1 cells, which are cell lines of epithelial cells, were cultured in DMEM/F-12 medium containing 10% FBS. cDNA of ARIA or cDNAs of ARIA-mKG-N and PTEN-mKG-C were incorporated into vectors, and the vectors were introduced into subconfluent CHO-K1 cells by a lipofection method. ARIA-mKG and PTEN-mKG were produced using a CoralHue Fluo-chase Kit (produced by Medical & Biological Laboratories (MBL)). The vectors contained a neomycin resistance gene. Twenty-four hours later, the cells were diluted 20 to 30 times and replated. Another 24 hours later, neomycin was added to the medium. Thereafter, the medium was replaced every two days and culturing continued until only the cells that stably expressed genes on the vectors survived and colonies derived from a single cell appeared. The cells in the colony were individually collected using a cloning cup, and monoclonal cell lines were isolated. Stable expression of the target proteins was confirmed by western blotting. Fig. 10 shows the results of western blotting performed on the cell lines that stably expressed ARIA. Stable line #6, which showed the highest expression of ARIA, was used in subsequent experiments.

From the above-described procedure, cell lines that express ARIA, and cell lines that simultaneously express ARIA-mKG and PTEN-mKG, thus enabling detection of a decrease in the function of either or both of ARIA and PTEN, were obtained.

Since CHO-K1 cells do not originally express ARIA, functional changes of ARIA-dependent PTEN can be made at a higher sensitivity by introducing the ARIA gene into CHO-K1 cells. ARIA, when expressed, binds to PTEN, and anchors PTEN to a plasma membrane. Thus, it becomes possible to perform screening of a substance that affects ARIA function by the following steps of: producing a substance that affects the binding between ARIA and PTEN; allowing the substance to act on ARIA expression cell lines; isolating membrane fractions; and measuring the PTEN expression level. These cell lines can be preferably used for secondary screening.

By using the cell lines that express ARIA-mKG-N and PTEN-mKG-C, the amount of bound ARIA-mKG-N and PTEN-mKG-C can be visually detected, and quantitative evaluation can be performed simply by using a plate reader. Such cell lines can thus be preferably used in screening for a substance that increases or decreases the binding of ARIA and PTEN, and are particularly suitable for preliminary screening due to their ease of operation.

By using these cell lines, it became possible to screen a substance that decreases or increases the function of ARIA.

### Example 4

### Mechanism of resistance to high-fat induced obesity in ARIA knockout mice

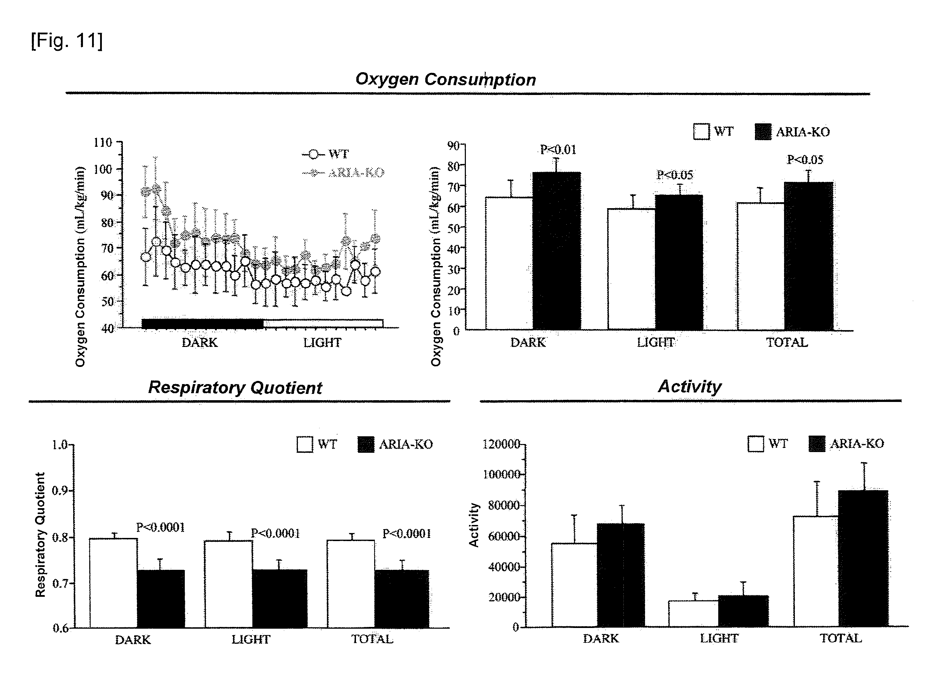

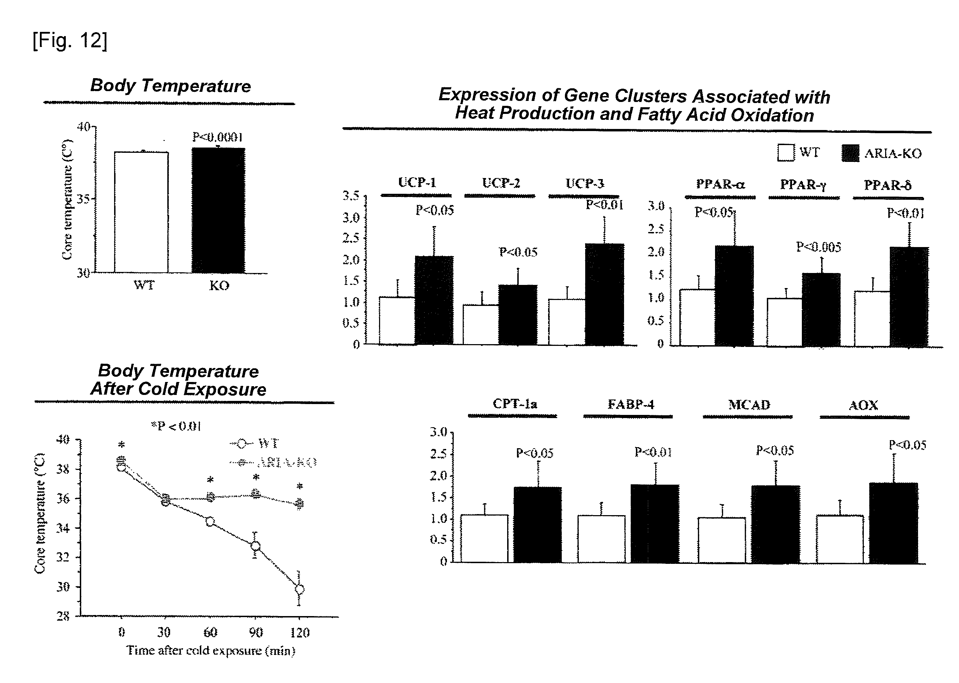

The present inventors discovered that ARIA knockout mice on a high-fat diet consumed more oxygen and showed a lower respiratory quotient than wild-type mice in spite of there being no difference in activity level (Fig. 11). This indicates that ARIA knockout mice consume energy at a high rate and use more fat as an energy source. The present inventors have also found that ARIA knockout mice on a high-fat diet had significantly higher body temperatures than wild-type mice, and the difference in body temperature became more noticeable by exposure to cold (Fig. 12). Heat production occurs in brown adipose through consumption of a large amount of aliphatic acid and energy. In the brown adipose of ARIA knockout mice on a high-fat diet, the expression of gene clusters associated with heat production and fatty acid oxidation was significantly increased (Fig. 12).

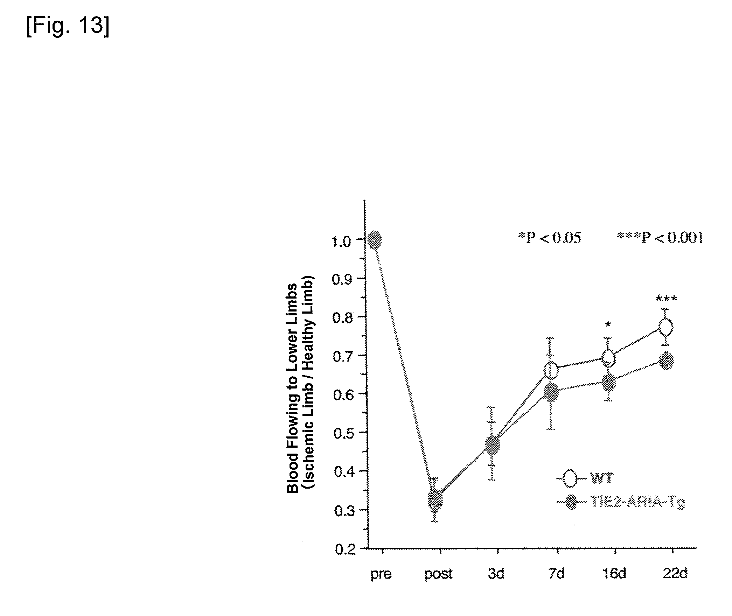

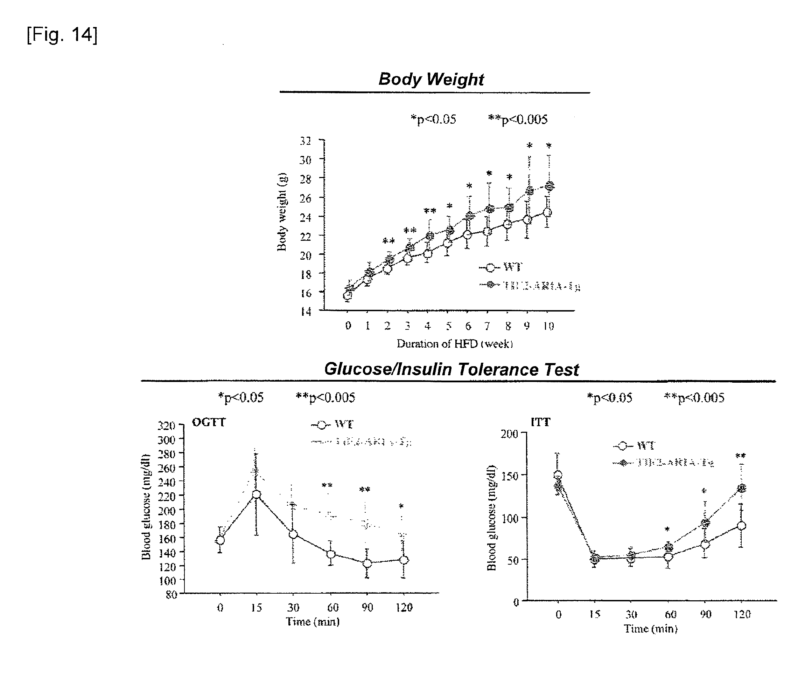

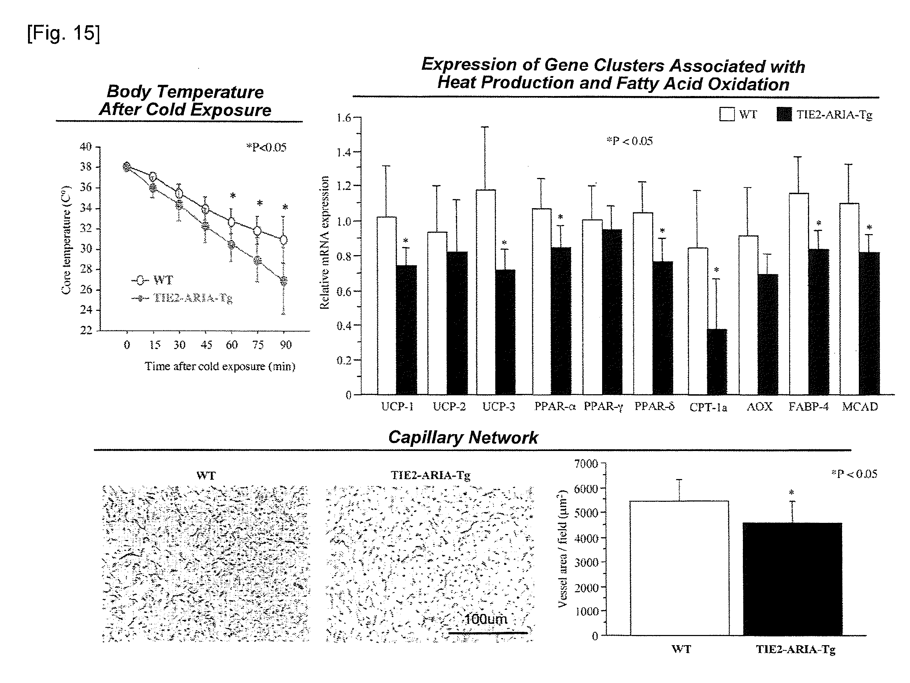

From the above-described findings, it is inferred that an increase in energy consumption leads to obesity resistance in ARIA knockout mice. In order to confirm this, the present inventors produced mice that overexpress ARIA specifically in vascular endothelial cells (TIE2-ARIA-Tg). It was confirmed that ischemia-induced angiogenesis was significantly reduced in TIE2-ARIA-Tg mice (Fig. 13). It was found that, unlike ARIA knockout mice, when fed a high-fat diet, TIE2-ARIA-Tg mice became susceptible to gaining weight, and glucose tolerance and insulin sensitivity worsened to a greater degree than wild-type mice (Fig. 14). In contrast to ARIA knockout mice, TIE2-ARIA-Tg mice on a high-fat diet showed a decrease in heat production capacity, and also showed a decrease in vascular density in brown adipose and a decrease in the expression of gene clusters associated with heat production and fatty acid oxidation (Fig. 15). The results suggested that functional inhibition of ARIA becomes a totally new target for developing a drug that increases energy consumption by promoting the proliferation of blood vessels, thereby controlling obesity.

## Claims

1. A cell for searching for an agent for prevention or treatment of a disease attributed to a decrease in insulin sensitivity or an obesity-controlling substance, or for searching for an obesity-inducing substance, the cell comprising a reporter gene under control of an ARIA gene promoter.

2. A cell **characterized by** an overexpression of ARIA by introduction of an ARIA gene.

3. The cell according to Claim 2, wherein the overexpression of ARIA enhances binding between ARIA and PTEN.

4. A cell for searching for an agent for prevention or treatment of a disease attributed to a decrease in insulin sensitivity or an obesity-controlling substance, or for searching for an obesity-inducing substance, the cell capable of detecting binding between an ARIA protein and a PTEN protein.

5. The cell according to Claim 4, wherein the ARIA protein and the PTEN protein each have been fused with a protein that produces or quenches fluorescence when the ARIA and PTEN proteins interact with each other.

6. An animal model of a disease attributed to a decrease in insulin sensitivity, the animal model **characterized by** an overexpression of ARIA gene.

7. The animal model according to Claim 6, which is a transgenic mouse.

8. A method for screening an agent for prevention or treatment of a disease attributed to a decrease in insulin sensitivity, or an obesity-controlling substance, an obesity-inducing substance or a non-human animal with a predisposition to obesity, the method using an increase and/or a decrease in an expression level of an ARIA gene as an index.

9. The method for screening an agent for prevention or treatment of a disease attributed to a decrease in insulin sensitivity or an obesity-controlling substance according to Claim 8, the method comprising:
contacting a candidate substance with the cell according to any one of Claims 1 to 5; and
determining the candidate substance to be an agent for prevention or treatment of a disease attributed to a decrease in insulin sensitivity or an obesity-controlling substance when the expression level of the ARIA gene is decreased.

10. The method for screening an obesity-inducing substance according to Claim 8, the method comprising:
contacting a candidate substance with the cell according to any one of Claims 1 to 5;
determining the candidate substance to be an obesity-inducing substance when the expression level of the ARIA gene is increased.

11. A method for screening an agent for prevention or treatment of a disease attributed to a decrease in insulin sensitivity, or an obesity-controlling substance or an obesity-inducing substance, the method comprising the steps of:
contacting a candidate substance with the animal model according to Claim 6 or 7; and
evaluating a change in insulin sensitivity.

12. A method for screening a nonhuman animal with a predisposition to obesity, the method comprising the steps of:
measuring an expression level of an ARIA gene in a nonhuman animal; and
determining the nonhuman animal to have a predisposition to obesity when the expression level of the ARIA gene is high.

13. An animal model for searching for an obesity-inducing substance, the animal model having an ARIA gene knocked out.

14. A method for detecting a disease attributed to a decrease in insulin sensitivity, the method comprising:
detecting an expression level of an ARIA protein in a subject sample; and
evaluating insulin sensitivity by using the expression level of the ARIA protein as an index.

15. The method according to Claim 14, wherein the sample is selected from the group consisting of blood, blood serum, blood plasma, urine, lachrymal fluid, and saliva, and preferably blood plasma or blood serum.
